# EUROPÄISCHE PATENTSCHRIFT

(11) **EP 0 640 063 B1**
(45) Veröffentlichungstag und Bekanntmachung des Hinweises auf die Patenterteilung: **22.11.1995**
(21) Anmeldenummer: 93909880.2
(22) Anmeldetag: 03.05.1993
(51) Int. Cl.: C07C 19/08, C07C 17/26

(54) **VERFAHREN ZUR HERSTELLUNG VON HEXAFLUORBUTAN**
PROCESS FOR PREPARING HEXAFLUOROBUTANE
PROCEDE DE PREPARATION D'HEXAFLUOROBUTANE

(30) Priorität: 14.05.1992 DE 4215876
(43) Veröffentlichungstag der Anmeldung: 01.03.1995
(73) Patentinhaber: BAYER AG, 51368 Leverkusen (DE)
(72) Erfinder: BIELEFELDT, Dietmar, D-4030 Ratingen 6 (DE); MARHOLD, Albrecht, D-5090 Leverkusen (DE)
(86) Internationale Anmeldenummer: EP9301073
(87) Internationale Veröffentlichungsnummer: WO9323354

(56) Entgegenhaltungen:
- EP-A- 0 442 087
- EP-A- 0 499 984

## Beschreibung

Die vorliegende Erfindung betrifft ein verbessertes Verfahren zur Herstellung von 1,1,1,4,4,4-Hexafluorbutan, d.h. von einem gesättigten, fluorhaltigen und chlorfreien Kohlenwasserstoff, wie er neuerdings als Treibmittel für Polyurethan-Schaumstoffe (als Ersatz für FCKW) von Interesse ist. Aus Chem. Lett. 1990, 879 bis 880 ist bekannt, daß bei der Verbindung CCl₂F-CClF₂ in Gegenwart von Wasserstoff an einem Nickel-Katalysator eine intramolekulare Chlorwasserstoffabspaltung unter Bildung der ungesättigten, chlorhaltigen Verbindung CClF=CF₂ stattfindet. Eine Dimerisierung zu Butan- oder Buten-Derivaten findet nicht statt.

Aus Chem. Lett. 1991, 1825 bis 1826 ist bekannt, daß sich aus der Verbindung CF₃-CCl₃ in Gegenwart von Wasserstoff und Argon als Verdünnungsmittel an einem Nickel-Katalysator durch Dimerisierung chlorhaltige Butene bilden, Aus der Verbindung CF₃-CFCl₂ entsteht unter diesen Bedingungen CF₃-CHClF neben geringen Anteilen eines perhalogenierten Butanderivats.

Es wurde nun ein Verfahren zur Herstellung von 1,1,1,4,4,4-Hexafluorbutan gefunden, das dadurch gekennzeichnet ist, daß man eine Trifluorethanverbindung der Formel (I)

CF₃-CXYZ (I),

in der
- X und Y: unabhängig voneinander für Wasserstoff, Chlor oder Brom und
- Z: für Chlor oder Brom stehen,
mit Wasserstoff in der Gasphase und ohne Verdünnungsmittel an einem palladium- und/oder nickelhaltigen Trägerkatalysator umsetzt und das Reaktionsprodukt, gegebenenfalls nach Abtrennung von 1,1,1,4,4,4-Hexafluorbutan, nachhydriert.

Die für das erfindungsgemäße Verfahren als Ausgangsverbindungen benötigten Trifluorethanverbindungen der Formel (I) sind gut zugänglich und Handelsprodukte.

Bevorzugt wird in das erfindungsgemäße Verfahren CF₃-CCl₃, CF₃-CHCl₂ oder CF₃-CH₂Br eingesetzt.

Die Umsetzung der Trifluorethanverbindungen der Formel (I) mit Wasserstoff kann z.B. so erfolgen, daß man die Verbindung der Formel (I) verdampft und gasförmig gemeinsam mit Wasserstoff über den palladium- und/oder nickelhaltigen Trägerkatalysator leitet. Bezogen auf 100 g der Verbindung der Formel (I) kann man z.B. 5 bis 200 Nl Wasserstoff einsetzen. Bevorzugt beträgt diese Menge 10 bis 30 Nl Wasserstoff.

Die Umsetzung von Verbindungen der Formel (I) mit Wasserstoff wird in Abwesenheit von Verdünnungsmitteln (z.B. Argon oder Stickstoff) durchgeführt.

Als Katalysatoren für diese Umsetzung kommen beispielsweise solche in Frage, die 0,5 bis 20 g Palladium und/oder 100 bis 1000 g Nickel pro Liter enthalten. Vorzugsweise enthalten die Katalysatoren 1 bis 10 g Palladium oder 300 bis 800 g Nickel pro Liter. Das Palladium und/oder das Nickel liegen dabei vorzugsweise vollständig, mindestens jedoch teilweise in elementarer Form vor. Als Trägermaterialien für diese Katalysatoren kommen z.B. Siliciumdioxid, Silikate, Aluminiumoxid, Spinelle, Bariumsulfat, Titandioxid, Magnesiumoxid und Kohle in Frage. Bevorzugt sind Siliciumdioxid und Aktiv-Kohlen, insbesondere Siliciumdioxid für Nickel und Aktiv-Kohlen für Palladium.

Gegebenenfalls können die Katalysatoren weitere Metalle als Promotoren enthalten. Promotoren können z.B. in Mengen von 0 bis 5 Gew.-%, bezogen auf Palladium bzw. Nickel vorhanden sein. Beispielsweise kann es sich bei den Promotoren um Zirkon, Titan, Vanadin, Niob, Tantal, Thallium, Zinn und/oder Kupfer handeln.

Die Umsetzung der Verbindungen der Formel (I) mit Wasserstoff kann bei Normaldruck oder erhöhtem Drucken, beispielsweise bis zu 20 bar, durchgeführt werden. Vorzugsweise arbeitet man bei Normaldruck. Die Temperatur für diese Umsetzung kann beispielsweise im Bereich 20 bis 550°C liegen. Bevorzugt sind Temperaturen zwischen 120 und 480°C, insbesondere solche von 250 bis 450°C.

Das nach dieser Umsetzung vorliegende Gasgemisch kann man beispielsweise aufarbeiten, indem man die organischen Bestandteile kondensiert und einer Nachhydrierung zuführt. Es ist bevorzugt, vor der Nachhydrierung aus dem kondensierten organischen Material bereits darin enthaltenes 1,1,1,4,4,4-Hexafluorbutan abzutrennen, z.B. durch Destillation.

Die Nachhydrierung des organischen Kondensates, das vorzugsweise kein 1,1,1,4,4,4-Hexafluorbutan mehr enthält, kann auf die gleiche Weise durchgeführt werden, wie die zuvor beschriebene Umsetzung der Verbindungen der Formel (I) mit Wasserstoff. Im Prinzip kann die Nachhydrierung auch entsprechend üblichen Hydrierungen des Standes der Technik durchgeführt werden, d.h. es können alle bekannten Hydrierkatalysatoren unter den jeweils bekannten Reaktionsbedingungen eingesetzt werden.

Es ist ausgesprochen überraschend, daß es mit dem erfindungsgemäßen Verfahren gelingt, 1,1,1,4,4,4-Hexafluorbutan in guten Ausbeuten zu erhalten, da dieses Produkt bei den eingangs geschilderten Verfahren des Standes der Technik nicht gebildet wird.

### Beispiele

### Beispiel 1

93,5 g/h 1,1,1-Trifluor-2,2,2-trichlorethan und 22 Nl/h Wasserstoff wurden bei einer Temperatur von 400°C über 200 ml eines Katalysators geleitet, der 5 g Palladium auf 1 l Aktivkohle enthielt. Hinter dem Katalysator wurde der Gasstrom auf -78°C abgekühlt und so im Verlauf von 1 Stunde 48 g rohes Produkt erhalten, Gemäß gaschromatographischer Analyse enthielt dieses Produkt 17,6 Gew.-% 1,1,1,4,4,4-Hexafluorbutan. Aus dem Gemisch wurden die leichter als 1,1,1,4,4,4-Hexafluorbutan flüchtigen Komponenten abgetrennt und unter den zuvor genannten Bedingungen erneut über den Katalysator geleitet. Mit einer Selektivität von 97 %, wurde bei dieser Nachhydrierung 1,1,1,4,4,4-Hexafluorbutan erhalten.

### Beispiel 2

38,5 g/h 1,1,1-Trifluor-2,2,2-trichlorethan und 11 Nl/h Wasserstoff wurden bei einer Temperatur von 300°C über 200 ml eines Katalysators geleitet, der 5 g Palladium auf 1 l Aktivkohle enthielt, Hinter dem Katalysator wurde der Gasstrom auf -78°C abgekühlt und so im Verlauf von 1 Stunde 3,4 g rohes Produkt erhalten, Gemäß gaschromatographischer Analyse enthielt dieses Produkt 11 Gew.-% 1,1,1,4,4,4-Hexafluorbutan. Aus dem Gemisch wurden die leichter als 1,1,1,4,4,4-Hexafluorbutan flüchtigen Komponenten abgetrennt und unter den zuvor genannten Bedingungen erneut über den Katalysator geleitet. Mit einer Selektivität von 98 %. wurde bei dieser Nachhydrierung 1,1,1,4,4,4-Hexafluorbutan erhalten.

### Beispiel 3

99,2 g/h 1,1,1-Trifluor-2,2,2-trichlorethan und 22 Nl/h Wasserstoff wurden bei einer Temperatur von 300°C über 200 ml eines Katalysators geleitet, der 500 g Nickel dotiert mit 0,5 Gew.-% Zirkon auf 1 l Siliciumdioxid enthielt. Hinter dem Katalysator wurde der Gasstrom auf -78°C abgekühlt und so im Verlauf von 1 Stunde 74 g rohes Produkt erhalten. Gemäß gaschromatographischer Analyse enthielt dieses Produkt 7 Gew.-% 1,1,1,4,4,4-Hexafluorbutan. Aus dem Gemisch wurden die leichter als 1,1,1,4,4,4-Hexafluorbutan flüchtigen Komponenten abgetrennt und unter den zuvor genannten Bedingungen erneut über den Katalysator geleitet. Mit einer Selektivität von 94 %. wurde bei dieser Nachhydrierung 1,1,1,4,4,4-Hexafluorbutan erhalten.

### Beispiel 4

Es wurde verfahren wie in Beispiel 3, jedoch wurden nur 94,4 g/h 1,1,1-Trifluor-2,2,2-trichlorethan eingesetzt. Es wurden 52 g rohes Produkt erhalten, das 38 Gew.-% 1,1,1,4,4,4-Hexafluorbutan enthielt. Die Nachhydrierung lieferte 1,1,1,4,4,4-Hexafluorbutan mit einer Selektivität von 93 %.

### Beispiel 5

66 g/h 1,1,1-Trifluor-2-bromethan und 11 Nl/h Wasserstoff wurden bei einer Temperatur von 400°C über 200 ml eines Katalysators geleitet, der 8 g Nickel auf 1 l Aktivkohle enthielt. Hinter dem Katalysator wurde der Gasstrom auf -78°C abgekühlt und so im Verlauf von 1 Stunde 10 g rohes Produkt erhalten. Gemäß gaschromatographischer Analyse enthielt dieses Produkt 6 Gew.-% 1,1,1,4,4,4-Hexafluorbutan. Aus dem Gemisch wurden die leichter als 1,1,1,4,4,4-Hexafluorbutan flüchtigen Komponenten abgetrennt und unter den zuvor genannten Bedingungen erneut über den Katalysator geleitet. Mit einer Selektivität von 98 % wurde bei dieser Nachhydrierung 1,1,1,4,4,4-Hexafluorbutan erhalten.

## Patentansprüche

1. Verfahren zur Herstellung von 1,1,1,4,4,4-Hexafluorbutan, dadurch gekennzeichnet, daß man eine Trifluorethanverbindung der Formel (I)
CF₃-CXYZ (I),
in der
X und Y unabhängig voneinander für Wasserstoff, Chlor oder Brom und
Z für Chlor oder Brom stehen,
mit Wasserstoff in der Gasphase und ohne Verdünnungsmittel an einem palladium- und/oder nickelhaltigen Trägerkatalysator umsetzt und das Reaktionsprodukt, gegebenenfalls nach Abtrennung von 1,1,1,4,4,4-Hexafluorbutan, nachhydriert.

2. Verfahren nach Anspruch 1, dadurch gekennzeichnet, daß man CF₃-CCl₃, CF₃-CHCl₂ oder CF₃-CH₂Br in das Verfahren einsetzt.

3. Verfahren nach Ansprüchen 1 und 2, dadurch gekennzeichnet, daß man bezogen auf 100 g der Verbindung der Formel (I) 5 bis 200 Nl Wasserstoff einsetzt.

4. Verfahren nach Ansprüchen 1 bis 3, dadurch gekennzeichnet, daß der Katalysator 0,5 bis 20 g Palladium und/oder 100 bis 1000 g Nickel pro Liter enthält.

5. Verfahren nach Ansprüchen 1 bis 4, dadurch gekennzeichnet, daß man es bei Temperaturen im Bereich 20 bis 550°C durchführt.

6. Verfahren nach Ansprüchen 1 bis 5, dadurch gekennzeichnet, daß man vor der Nachhydrierung 1,1,1,4,4,4-Hexafluorbutan aus dem Reaktionsprodukt abtrennt.

7. Verfahren nach Ansprüchen 1 bis 6, dadurch gekennzeichnet, daß man die Nachhydrierung in gleicher Weise durchführt wie die Umsetzung der Verbindung der Formel (I) mit Wasserstoff.

## Claims

1. A process for preparing 1,1,1,4,4,4-hexafluorobutane, characterised in that a trifluoroethane compound of the formula (I)
CF₃-CXYZ (I)
in which
X and Y independently of each other represent hydrogen, chlorine or bromine and
Z represents chlorine or bromine,
is reacted with hydrogen in the gas phase and without a diluent on a palladium- and/or nickel-containing supported catalyst and the reaction product, optionally after separating out 1,1,1,4,4,4-hexafluorobutane, is subsequently hydrogenated.

2. A process according to Claim 1, characterised in that CF₃-CCl₃, CF₃-CHCl₂ or CF₃-CH₂Br is used in the process.

3. A process according to Claims 1 and 2, characterised in that 5 to 200 Nl of hydrogen are used for each 100 g of the compound of formula (I).

4. A process according to Claims 1 to 3, characterised in that the catalyst contains 0.5 to 20 g of palladium and/or 100 to 1000 g of nickel per litre.

5. A process according to Claims 1 to 4, characterised in that it is performed at temperatures in the range 20 to 550°C.

6. A process according to Claims 1 to 5, characterised in that 1,1,1,4,4,4-hexafluorobutane is separated from the reaction product before subsequent hydrogenation.

7. A process according to Claims 1 to 6, characterised in that the subsequent hydrogenation is performed in the same way as the reaction of the compound of formula (I) with hydrogen.

## Revendications

1. Procédé de préparation du 1,1,1,4,4,4-hexafluorobutane, caractérisé en ce que l'on fait réagir un dérivé du trifluoréthane répondant à la formule (I)
CF₃-CXYZ (I),
dans laquelle
X et Y représentent chacun, indépendamment l'un de l'autre, l'hydrogène, le chlore ou le brome et
Z représente le chlore ou le brome,
avec l'hydrogène en phase gazeuse, en l'absence de diluant, sur un catalyseur au palladium et(ou au nickel sur support, et on soumet le produit de réaction, éventuellement après séparation du 1,1,1,4,4,4-hexafluorobutane, à une hydrogénation complémentaire.

2. Procédé selon la revendication 1, caractérisé en ce que l'on met en oeuvre dans le procédé CF₃-CCl₃, CF₃-CHCl₂ ou CF₃-CH₂Br.

3. Procédé selon les revendications 1 et 2, caractérisé en ce que l'on utilise de 5 à 200 l normaux d'hydrogène pour 100 g du composé de formule (I).

4. Procédé selon les revendications 1 à 3, caractérisé en ce que le catalyseur contient 0,5 à 20 g de palladium et/ou 100 à 1000 g de nickel par litre.

5. Procédé selon les revendications 1 à 4, caractérisé en ce que l'on opère à des températures dans l'intervalle de 20 à 550°C.

6. Procédé selon les revendications 1 à 5, caractérisé en ce que, avant l'hydrogénation complémentaire, on sépare le 1,1,1,4,4,4-hexafluorobutane du produit de réaction.

7. Procédé selon les revendications 1 à 6, caractérisé en ce que l'on réalise l'hydrogénation complémentaire dans les mêmes conditions que la réaction du composé de formule (I) avec l'hydrogène.
